# EUROPEAN PATENT SPECIFICATION

(11) **EP 4 159 711 B1**
(45) Date of publication and mention of the grant of the patent: **25.06.2025**
(21) Application number: 21944454.4
(22) Date of filing: 30.08.2021
(51) Int. Cl.: H01B 3/18, C07C 69/60, C07C 67/08, C08K 5/134

(54) **CROSS-LINKED POLYETHYLENE INSULATING MATERIAL CONTAINING A MALEIC ANHYDRIDE MODIFIED VOLTAGE STABILIZER**
VERNETZTES POLYETHYLEN-ISOLIERMATERIAL ENTHALTEND EINEN MALEINSÄUREANHYDRIDMODIFIZIERTEN SPANNUNGSSTABILISATOR
MATÉRIAU ISOLANT EN POLYÉTHYLÈNE RÉTICULÉ CONTENANT UN STABILISATEUR DE TENSION MODIFIÉ PAR UN ANHYDRIDE MALÉIQUE

(30) Priority: 19.08.2021 CN 202110957274
(43) Date of publication of application: 05.04.2023
(73) Proprietor: Electric Power Research Institute. China Southern Power Grid, Guangzhou, Guangdong 510663 (CN); Shenzhen Power Supply Co., Ltd., Shenzhen, Guangdong 518001 (CN)
(72) Inventor: HOU, Shuai, Guangzhou Guangdong 510663 (CN); FU, Mingli, Guangzhou Guangdong 510663 (CN); LI, Xiaolin, Guangzhou Guangdong 510663 (CN); ZHU, Wenbo, Guangzhou Guangdong 510663 (CN); HUI, Baojun, Guangzhou Guangdong 510663 (CN); FENG, Bin, Guangzhou Guangdong 510663 (CN); ZHANG, Yifan, Guangzhou Guangdong 510663 (CN); ZHAN, Yunpeng, Guangzhou Guangdong 510663 (CN); CHEN, Xiao, Guangzhou Guangdong 510663 (CN); ZHANG, Bin, Guangzhou Guangdong 510663 (CN); XU, Shu, Guangzhou Guangdong 510663 (CN); WU, Guoxing, Guangzhou Guangdong 510663 (CN)
(74) Representative: Hoffmann Eitle
(86) International application number: PCT/CN2021/115234
(87) International publication number: WO 2023/019632

(56) References cited:
- EP-A2- 0 280 454
- WO-A1-2018/121657
- CN-A- 108 484 975
- CN-A- 109 776 910
- CN-A- 109 776 910
- CN-A- 111 825 798
- CN-A- 111 825 798
- FR-A1- 2 021 937

## Description

### TECHNICAL FIELD

The present invention relates to a cross-linked polyethylene AC cable insulating material comprising a maleic anhydride modified voltage stabilizer, a preparation method thereof, and use thereof, which belong to the technical field of high voltage and insulation.

### BACKGROUND

Cross-linked polyethylene insulating material has many advantages and is currently the most important insulating material for power cables. At present, there is a tendency for cross-linked polyethylene insulated power cables to replace the other cables at various voltage levels. However, the manufacturing process of the cross-linked polyethylene insulating material inevitably introduces impurities, whose amount and size significantly affect working life and operating stability of the cables made of the insulating material. Electrical treeing would easily occur due to the impurities when the cross-linked polyethylene insulating material is used under a strong alternating electric field, causing quickly and early breakdown of the insulating layers of the cables. In most situations, the breakdown of the insulating layers due to electrical tree makes the working life of the cables far less than expected.

In order to improve the working stability and lifetime of the cross-linked polyethylene insulated cables, the most commonly used method in the field is to modify the insulating material. The modification of the cross-linked polyethylene using a voltage stabilizer has a long history. Until now a variety of voltage stabilizers have emerged.

CN109776910A discloses a cross-linked polyethylene insulating material for a high voltage direct current cable, whose preparation involves treating low-density polyethylene with dicumyl peroxide and 4,4'-dihydroxybenzophenone.

Generally, the use of the voltage stabilizer can significantly improve the resistance to electrical treeing in the cross-linked polyethylene insulator, increasing the inception voltage of electrical tree and prolonging the time from the electrical treeing inception to the breakdown of the material.

However, the voltage stabilizers in prior art are usually small molecule additives containing benzene rings and having a certain polarity. Within the range of their effective addition amount, the voltage stabilizers in prior art would lead to increase in dielectric loss tangent and decrease in electrical resistivity of the insulating material. As an insulating material for alternating current (AC) cables, the value of the dielectric loss tangent at 50 Hz has to be limited to a standard range, and the smaller the value is, the better the transmission capacity and performance of the AC cables are improved.

At present, relying on the existing technology, it is difficult to improve the resistance to electrical treeing by adding a voltage stabilizer without increasing the dielectric loss of the insulating material, which means that it is difficult to solve the contradiction between the electrical resistance performance and the dielectric loss performance. It is important and significant to design and synthesize a new voltage stabilizer, which, in its effective addition amount, can improve the resistance to electrical treeing, prolong the working life, and improve the insulation resistivity of the insulating material, and can further limit the value of the dielectric loss tangent of the insulating material to a standard range.

### SUMMARY

An object of the present invention is to overcome the deficiencies in prior art by using a maleic anhydride modified voltage stabilizer whose structural formula is represented by Formula 1: 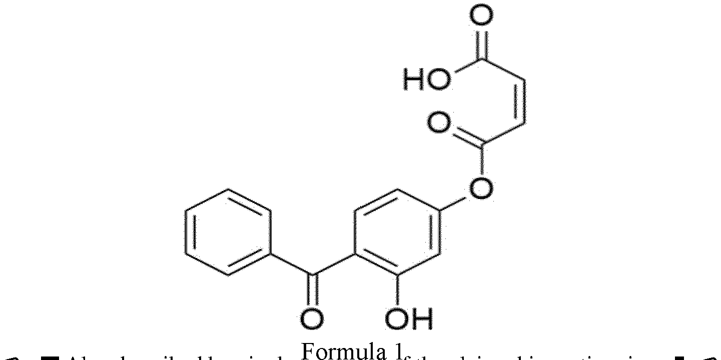 Also described nerein, but not part or the claimed invention, is

a method for preparing the maleic anhydride modified voltage stabilizer, including following steps:
(1) dissolving maleic anhydride and 2,4-dihydroxybenzophenone in tetrahydrofuran to obtain a mixture;
(2) in a protective gas atmosphere, adding a catalyst to the mixture obtained in step (1), stirring, adding water and centrifuging, and then drying an obtained precipitate, thereby achieving the maleic anhydride modified voltage stabilizer;
wherein a molar ratio of 2,4-dihydroxybenzophenone, maleic anhydride, tetrahydrofuran, and catalyst is 2:(1.4-2):(24-125):(0.1-1.2).

Preferably, the catalyst in step (2) is concentrated sulfuric acid or p-toluenesulfonic acid.

Preferably, in step (2), stirring time is 6-12 hours(h), and stirring temperature is 65-70°C.

The reaction scheme of the preparation method is as follows:

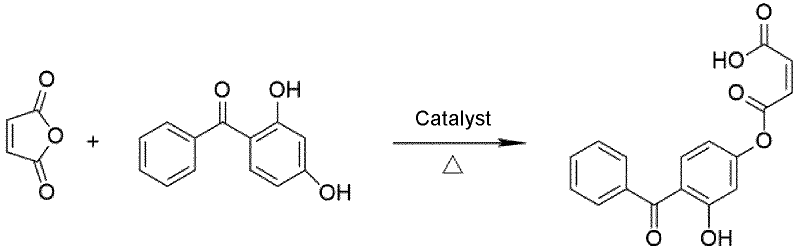

The present invention provides the use of the maleic anhydride modified voltage stabilizer in a cross-linked polyethylene AC cable insulating material.

In particular, the present invention provides a cross-linked polyethylene AC cable insulating material as defined in the appended claims, which comprises the maleic anhydride modified voltage stabilizer of formula I.

The cross-linked polyethylene AC cable insulating material of the present invention comprises the following components by mass: 100phr of low-density polyethylene, 0.2-1.2phr of a maleic anhydride modified voltage stabilizer, 0.2-0.5phr of an antioxidant, and 1.5-2.2phr of an initiator. The low-density polyethylene has a melt index of 1.9-2.1g/10min at 190°C and 2.16kg load and a density of 0.902-0.942g/cm³.

The maleic anhydride modified voltage stabilizer in the present application is used as a voltage stabilizer in the cross-linked polyethylene AC cable insulating material. The voltage stabilizer has graftable chemical activity and can be grafted onto the cross-linked polyethylene through free radical reaction caused by a cross-linking agent, preventing migration and precipitation of the voltage stabilizer, which can increase the inception voltage of the AC electrical treeing in the cross-linked polyethylene for a long term. As a traditional voltage stabilizer, 2,4-dihydroxybenzophenone can increase the inception voltage of the electrical treeing to a certain degree. However, 2,4-dihydroxybenzophenone as the voltage stabilizer can only be simply physically mixed with the cross-linked polyethylene. Moreover, 2,4-dihydroxybenzophenone is easy to precipitate, so that its long-term effect is difficult to guarantee. In addition, 2,4-dihydroxybenzophenone has a negative impact on both the insulation resistivity and the dielectric loss tangent of the cross-linked polyethylene AC cable insulating material, resulting in a decrease in resistivity and an increase in dielectric loss tangent. After long-term experimental exploration of various voltage stabilizers, the inventors found that grafting the voltage stabilizer to the polymer macromolecule via a chemical bond can significantly improve the migration resistance of the voltage stabilizer. In addition, the inventors also found that maleic anhydride has a chemical bond with a high polarity, which can introduce a deep charge trap into the polymer, thereby restricting migration and conduction of charge carriers under the electric field, and resulting in significant increase in the electrical resistivity of the cross-linked polyethylene AC cable insulating material.

As a preferred embodiment of the cross-linked polyethylene AC cable insulating material, the antioxidant is antioxidant 300.

As a preferred embodiment of the cross-linked polyethylene AC cable insulating material, the initiator is dicumyl peroxide.

Further, the present invention provides a method for preparing the cross-linked polyethylene AC cable insulating material, including following steps:
(1) melting and blending low-density polyethylene, the maleic anhydride modified voltage stabilizer, and an antioxidant together in an extruder, and then extruding and granulating resulting blend, thereby obtaining granules;
(2) adding an initiator melted at 70-80°C to the granules obtained in step (1) to infiltrate the granules, thereby obtaining a polyethylene material;
(3) cross-linking the polyethylene material obtained in step (2) at 150-280°C, 10-20MPa, thereby obtaining the cross-linked polyethylene AC cable insulating material.

Compared with prior art, the present invention has following beneficial effects. The present invention provides the maleic anhydride modified voltage stabilizer, which is used as a voltage stabilizer in the cross-linked polyethylene AC cable insulating material. In the range of effective addition amount, the maleic anhydride modified voltage stabilizer can improve the resistance to electrical treeing, prolong the working life, and improve the insulation resistivity of the insulating material, and can further limit the value of the dielectric loss tangent of the cross-linked polyethylene AC cable insulating material to a standard range.

### BRIEF DESCRIPTION OF THE DRAWINGS

FIG. 1 shows an infrared absorption spectrum of a maleic anhydride modified voltage stabilizer.
FIG. 2 shows a proton nuclear magnetic resonance spectrum of the maleic anhydride modified voltage stabilizer.
FIG. 3 shows infrared absorption spectra of cross-linked polyethylene AC cable insulating materials of Example 6 and Comparative Example 1.
FIG. 4 shows Weibull distribution plots of inception voltages of electrical treeing in cross-linked polyethylene AC cable insulating materials of Example 7 and Comparative Example 2.
FIG. 5 shows values of dielectric loss tangent of cross-linked polyethylene AC cable insulating materials of Examples 7 to 9 and Comparative Example 2.
FIG. 6 shows values of dielectric loss tangent of a cross-linked polyethylene AC cable insulating material of Comparative Example 3.
FIG. 7 shows electrical conductivities of cross-linked polyethylene AC cable insulating materials of Examples 7-9 and Comparative Example 2 at different strengths of electric field.
FIG. 8 shows a structural formula of the maleic anhydride modified voltage stabilizer.

### DETAILED DESCRIPTION

In order to better illustrate the objects, technical solutions and advantages of the present invention, the present invention will be further described below with reference to specific embodiments and drawings.

### Reference example 1

In a first aspect, the present example provides a maleic anhydride modified voltage stabilizer whose structural formula is represented by Formula 1:

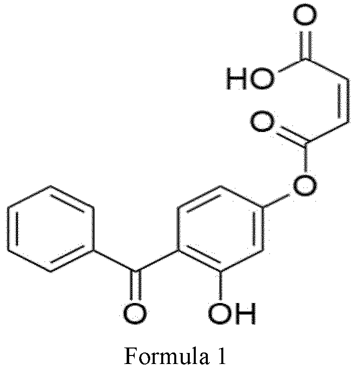

In a second aspect, the present example provides a method for preparing the maleic anhydride modified voltage stabilizer, including the following steps of:
(1) dissolving maleic anhydride and 2,4-dihydroxybenzophenone in tetrahydrofuran to obtain a mixture;
(2) in a nitrogen gas atmosphere, adding concentrated sulfuric acid to the mixture obtained in step (1), stirring at 70°C for 10h, adding water and centrifuging, and then drying an obtained precipitate at 80°C, thereby achieving the maleic anhydride modified voltage stabilizer;
wherein a molar ratio of 2,4-dihydroxybenzophenone, maleic anhydride, tetrahydrofuran, and catalyst (concentrated sulfuric acid) is 2:1.4:24:0.1.

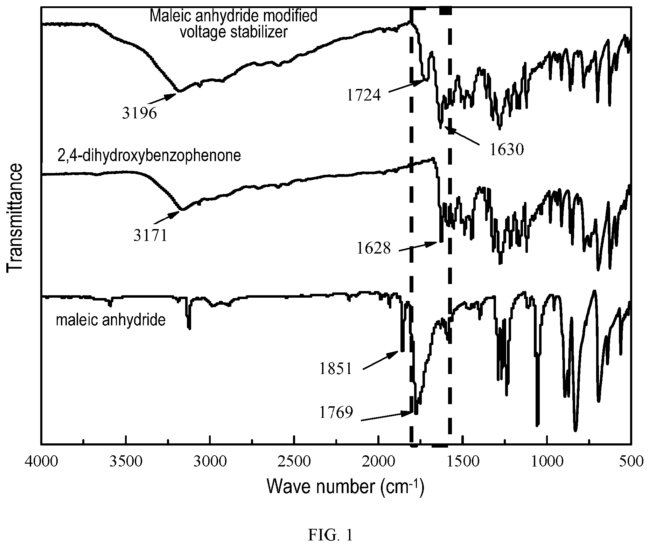

FIG. 1 shows an infrared absorption spectrum of the maleic anhydride modified voltage stabilizer. It can be seen from FIG. 1 that the absorption peaks at 1851 cm⁻¹ and 1769 cm⁻¹, respectively, correspond to asymmetric and symmetric stretching vibration absorption of C=O in maleic anhydride. Many similar characteristic peaks can be observed in the spectra of 2,4-dihydroxybenzophenone and the maleic anhydride modified voltage stabilizer. Except the range of 1600-1450 cm⁻¹ corresponding to benzene ring skeleton vibration absorption bonds, the peaks at 3171 cm⁻¹ and 1628 cm⁻¹ in the spectrum of 2,4-dihydroxybenzophenone and at 3196 cm⁻¹ and 1630 cm⁻¹ in the spectrum of the maleic anhydride modified voltage stabilizer are attributed to -OH and C=O which are connected with benzene ring. In addition, the spectrum of the maleic anhydride modified voltage stabilizer has a new peak (1724 cm⁻¹) corresponding to the ester group C=O, indicating that maleic anhydride was successfully esterified with 2,4-dihydroxybenzophenone.

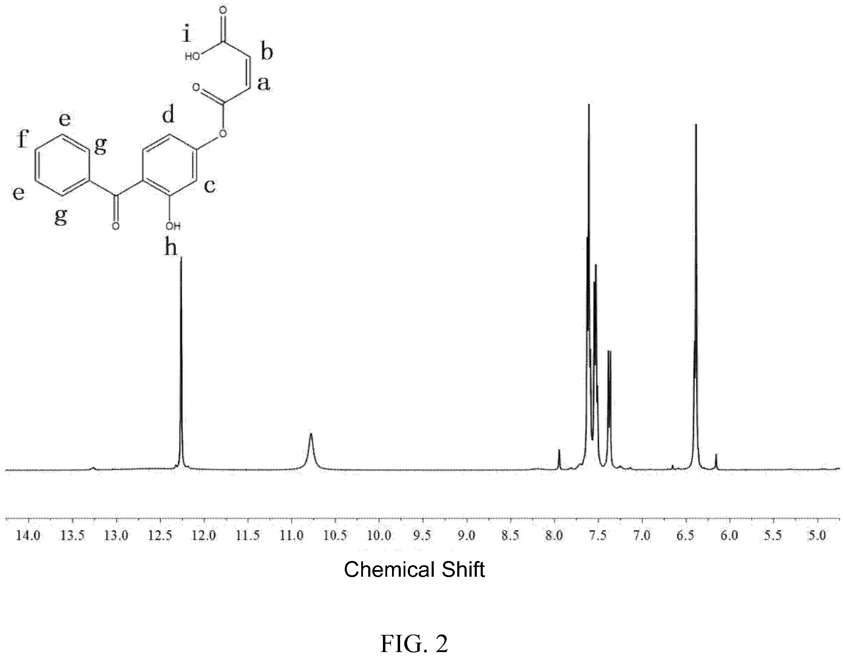

FIG. 2 shows a proton nuclear magnetic resonance spectrum of the maleic anhydride modified voltage stabilizer. It can be known from FIG. 2 that the peaks between 6.38 ppm and 6.40 ppm are attributed to the chemical shifts of the acid anhydride protons (-CH=CH-). The aromatic protons of the benzene rings resonate around 7.38, 7.50, 7.53, 7.59, 7.61 and 7.63 ppm, and the peaks around 10.75 and 12.35 ppm belong to O-H. The integrated intensity ratio of the peaks is 1:1:1:1:1:1:1:1:2:1:2:1:1:1, which is completely consistent with the theoretical value predicted according to structure calculation, confirming that the maleic anhydride modified voltage stabilizer has been successfully synthesized.

### Reference example 2

In a first aspect, the present example provides a maleic anhydride modified voltage stabilizer whose structural formula is represented by Formula 1:

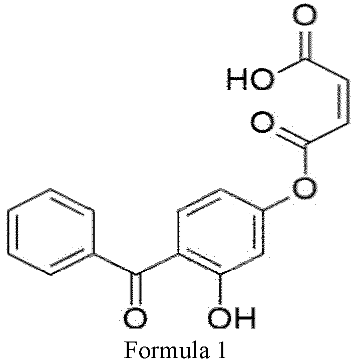

In a second aspect, the present example provides a method for preparing the maleic anhydride modified voltage stabilizer, including the following steps of:
(1) dissolving maleic anhydride and 2,4-dihydroxybenzophenone in tetrahydrofuran to obtain a mixture;
(2) in a nitrogen gas atmosphere, adding concentrated sulfuric acid to the mixture obtained in step (1), stirring at 68°C for 6h, adding water and centrifuging, and then drying an obtained precipitate at 80°C, thereby achieving the maleic anhydride modified voltage stabilizer;
wherein a molar ratio of 2,4-dihydroxybenzophenone, maleic anhydride, tetrahydrofuran, and catalyst (concentrated sulfuric acid) is 2:1.7:60:0.5.

### Reference example 3

In a first aspect, the present example provides a maleic anhydride modified voltage stabilizer whose structural formula is represented by Formula 1:

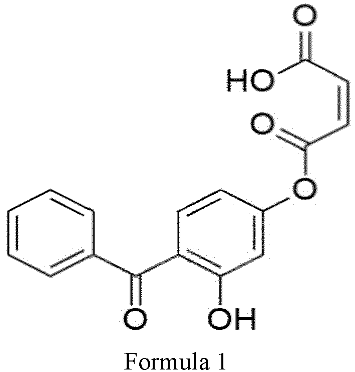

In a second aspect, the present example provides a method for preparing the maleic anhydride modified voltage stabilizer, including the following steps of:
(1) dissolving maleic anhydride and 2,4-dihydroxybenzophenone in tetrahydrofuran to obtain a mixture;
(2) in a nitrogen gas atmosphere, adding p-toluenesulfonic acid to the mixture obtained in step (1), stirring at 65°C for 12h, adding water and centrifuging, and then drying an obtained precipitate at 80°C, thereby achieving the maleic anhydride modified voltage stabilizer;
wherein a molar ratio of 2,4-dihydroxybenzophenone, maleic anhydride, tetrahydrofuran, and catalyst (p-toluenesulfonic acid) is 2:2:125:1.2.

### Example 4

The present example provides a cross-linked polyethylene AC cable insulating material, including the following components by mass: 100phr of low-density polyethylene, 0.2phr of a voltage stabilizer, 0.2phr of antioxidant 300, and 1.5phr of dicumyl peroxide. The low-density polyethylene has a melt index of 1.9g/10min at 190°C and 2.16kg load and a density of 0.902g/cm³. The voltage stabilizer is the maleic anhydride modified voltage stabilizer in Example 1.

The present example also provides a method for preparing the cross-linked polyethylene AC cable insulating material, including the following steps of:
(1) melting and blending 100phr of low-density polyethylene, 0.2phr of the maleic anhydride modified voltage stabilizer, and 0.2phr of antioxidant 300 together in an extruder at 110°C, and then extruding and granulating resulting blend;
(2) adding 1.5phr of dicumyl peroxide melted at 70°C to granules obtained in step (1) to infiltrate the granules, thereby obtaining a polyethylene material;
(3) cross-linking the polyethylene material obtained in step (2) at 150°C, 20MPa, thereby obtaining the cross-linked polyethylene AC cable insulating material.

### Example 5

The present example provides a cross-linked polyethylene AC cable insulating material, including the following components by mass: 100phr of low-density polyethylene, 1.2phr of a voltage stabilizer, 0.5phr of antioxidant 300, and 2.2phr of dicumyl peroxide. The low-density polyethylene has a melt index of 2.1g/10min at 190°C and 2.16kg load and a density of 0.942g/cm³. The voltage stabilizer is the maleic anhydride modified voltage stabilizer in Example 1.

The present example also provides a method for preparing the cross-linked polyethylene AC cable insulating material, including the following steps of:
(1) melting and blending 100phr of low-density polyethylene, 1.2phr of the maleic anhydride modified voltage stabilizer, and 0.5phr of antioxidant 300 together in an extruder at 135°C, and then extruding and granulating the resulting blend;
(2) adding 2.2phr of dicumyl peroxide melted at 80°C to granules obtained in step (1) to infiltrate the granules, thereby obtaining a polyethylene material;
(3) cross-linking the polyethylene material obtained in step (2) at 280°C, 10MPa, thereby obtaining the cross-linked polyethylene AC cable insulating material.

### Example 6

The present example provides a cross-linked polyethylene AC cable insulating material, including the following components by mass: 100phr of low-density polyethylene, 1.2phr of a voltage stabilizer, 0.4phr of antioxidant 300, and 2phr of dicumyl peroxide. The low-density polyethylene has a melt index of 2g/10min at 190°C and 2.16kg load and a density of 0.922g/cm³. The voltage stabilizer is the maleic anhydride modified voltage stabilizer in Example 1.

The present example also provides a method for preparing the cross-linked polyethylene AC cable insulating material, including the following steps of:
(1) melting and blending 100phr of low-density polyethylene, 2phr of the maleic anhydride modified voltage stabilizer, and 0.4phr of antioxidant 300 together in an extruder at 120°C, and then extruding and granulating the resulting blend;
(2) adding 2phr of dicumyl peroxide melted at 75°C to granules obtained in step (1) to infiltrate the granules, thereby obtaining a polyethylene material;
(3) cross-linking the polyethylene material obtained in step (2) at 175°C, 15MPa, thereby obtaining the cross-linked polyethylene AC cable insulating material, referred as Low-density polyethylene+DCP+Maleic anhydride modified voltage stabilizer (After cross-linking).

### Comparative Example 1

The present comparative example provides a cross-linked polyethylene AC cable insulating material, including the following components by mass: 100 phr of low-density polyethylene, 2phr of a voltage stabilizer, 0.4phr of antioxidant 300, and 2phr of dicumyl peroxide. The low-density polyethylene has a melt index of 2g/10min at 190°C and 2.16kg load and a density of 0.922g/cm³. The voltage stabilizer is the maleic anhydride modified voltage stabilizer in Example 1.

The present comparative example also provides a method for preparing the cross-linked polyethylene AC cable insulating material, including the following steps of:
(1) melting and blending 100phr of low-density polyethylene, 2phr of the maleic anhydride modified voltage stabilizer, and 0.4phr of antioxidant 300 together in an extruder at 120°C, and then extruding and granulating the resulting blend;
(2) adding 2phr of dicumyl peroxide melted at 75°C to granules obtained in step (1) to infiltrate the granules, thereby obtaining a polyethylene material, referred as Low-density polyethylene+DCP+Maleic anhydride modified voltage stabilizer (Before cross-linking).

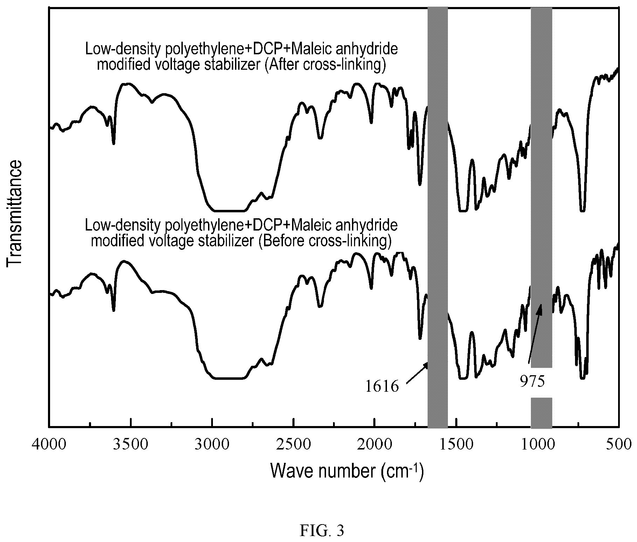

FIG. 3 shows infrared absorption spectra of the cross-linked polyethylene AC cable insulating materials of Example 6 and Comparative Example 1, which is used to illustrate the cross-linking reaction between the maleic anhydride modified voltage stabilizer and the polyethylene. As can be seen from FIG. 3, there are absorption peaks at 1616cm⁻¹ and 975cm⁻¹ in the infrared spectrum of the cross-linked polyethylene AC cable insulating material of Comparative Example 1, which are produced by unsaturated vinyl of the maleic anhydride modified voltage stabilizer. In the infrared spectrum of the cross-linked polyethylene AC cable insulating material of Example 6, the absorption peaks at 1616cm⁻¹ and 975cm⁻¹ have disappeared, indicating that during the cross-linking process, the free radicals in the unsaturated vinyl of the maleic anhydride modified voltage stabilizer are bonded to the macromolecular chain of the cross-linked polyethylene via addition reaction. Therefore, the maleic anhydride modified voltage stabilizer used as the voltage stabilizer of the cross-linked polyethylene AC cable insulating material is not easy to migrate or precipitate, and the voltage stabilization effect of the maleic anhydride modified voltage stabilizer can be maintained for a long time.

### Example 7

The present example provides a cross-linked polyethylene AC cable insulating material, including the following components by mass: 100phr of low-density polyethylene, 0.8phr of a voltage stabilizer, 0.4phr of antioxidant 300, and 2phr of dicumyl peroxide. The low-density polyethylene has a melt index of 2g/10min at 190°C and 2.16kg load and a density of 0.922g/cm³. The voltage stabilizer is the maleic anhydride modified voltage stabilizer in Example 1.

The present example also provides a method for preparing the cross-linked polyethylene AC cable insulating material, including the following steps of:
(1) melting and blending 100phr of low-density polyethylene, 0.8phr of the maleic anhydride modified voltage stabilizer, and 0.4phr of antioxidant 300 together in an extruder at 120°C, and then extruding and granulating the resulting blend;
(2) adding 2phr of dicumyl peroxide melted at 75°C to granules obtained in step (1) to infiltrate the granules, thereby obtaining a polyethylene material;
(3) cross-linking the polyethylene material obtained in step (2) at 170°C, 15MPa, thereby obtaining the cross-linked polyethylene AC cable insulating material, referred as XLPE+0.8phr maleic anhydride modified voltage stabilizer.

### Example 8

The present example provides a cross-linked polyethylene AC cable insulating material, including the following components by mass: 100phr of low-density polyethylene, 0.4phr of a voltage stabilizer, 0.4phr of antioxidant 300, and 2phr of dicumyl peroxide. The low-density polyethylene has a melt index of 2g/10min at 190°C and 2.16kg load and a density of 0.922g/cm³. The voltage stabilizer is the maleic anhydride modified voltage stabilizer in Example 1.

The present example also provides a method for preparing the cross-linked polyethylene AC cable insulating material, including the following steps of:
(1) melting and blending 100phr of low-density polyethylene, 0.4phr of the maleic anhydride modified voltage stabilizer, and 0.4phr of antioxidant 300 together in an extruder at 120°C, and then extruding and granulating the blend;
(2) adding 2phr of dicumyl peroxide melted at 75°C to granules obtained in step (1) to infiltrate the granules, thereby obtaining a polyethylene material;
(3) cross-linking the polyethylene material obtained in step (2) at 175°C, 15MPa, thereby obtaining the cross-linked polyethylene AC cable insulating material, referred as XLPE+0.4phr maleic anhydride modified voltage stabilizer.

### Example 9

The present example provides a cross-linked polyethylene AC cable insulating material, including the following components by mass: 100phr of low-density polyethylene, 1.2phr of a voltage stabilizer, 0.4phr of antioxidant 300, and 2phr of dicumyl peroxide. The low-density polyethylene has a melt index of 2g/10min at 190°C and 2.16kg load and a density of 0.922g/cm³. The voltage stabilizer is the maleic anhydride modified voltage stabilizer in Example 1.

The present example also provides a method for preparing the cross-linked polyethylene AC cable insulating material, including the following steps of:
(1) melting and blending 100phr of low-density polyethylene, 1.2phr of the maleic anhydride modified voltage stabilizer, and 0.4phr of antioxidant 300 together in an extruder at 120°C, and then extruding and granulating resulting blend;
(2) adding 2phr of dicumyl peroxide melted at 75°C to granules obtained in step (1) to infiltrate the granules, thereby obtaining a polyethylene material;
(3) cross-linking the polyethylene material obtained in step (2) at 175°C, 15MPa, thereby obtaining the cross-linked polyethylene AC cable insulating material, referred as XLPE+1.2phr maleic anhydride modified voltage stabilizer.

### Comparative Example 2

The present example provides a cross-linked polyethylene AC cable insulating material, including the following components by mass: 100phr of low-density polyethylene, 0.4phr of antioxidant 300, and 2phr of dicumyl peroxide. The low-density polyethylene has a melt index of 2g/10min at 190°C and 2.16kg load and a density of 0.922g/cm³.

The present example also provides a method for preparing the cross-linked polyethylene AC cable insulating material, including the following steps of:
(1) melting and blending 100phr of low-density polyethylene and 0.4phr of antioxidant 300 together in an extruder at 120°C, and then extruding and granulating the blend;
(2) adding 2phr of dicumyl peroxide melted at 75°C to granules obtained in step (1) to infiltrate the granules, thereby obtaining a polyethylene material;
(3) cross-linking the polyethylene material obtained in step (2) at 175°C, 15MPa, thereby obtaining the cross-linked polyethylene AC cable insulating material, referred as XLPE.

### Comparative Example 3

The present example provides a cross-linked polyethylene AC cable insulating material, including the following components by mass: 100phr of low-density polyethylene, 0.8phr of 2,4-dihydroxybenzophenone, 0.4phr of antioxidant 300, and 2phr of dicumyl peroxide. The low-density polyethylene has a melt index of 2g/10min at 190°C and 2.16kg load and a density of 0.922g/cm³.

The present example also provides a method for preparing the cross-linked polyethylene AC cable insulating material, including the following steps of:
(1) melting and blending 100phr of low-density polyethylene, 0.8phr of 2,4-dihydroxybenzophenone, and 0.4phr of antioxidant 300 together in an extruder at 120°C, and then extruding and granulating the blend;
(2) adding 2phr of dicumyl peroxide melted at 75°C to granules obtained in step (1) to infiltrate the granules, thereby obtaining a polyethylene material;
(3) cross-linking the polyethylene material obtained in step (2) at 175°C, 15MPa, thereby obtaining the cross-linked polyethylene AC cable insulating material, referred as XLPE+0.8phr 2,4-dihydroxybenzophenone.

### Effect Example 1

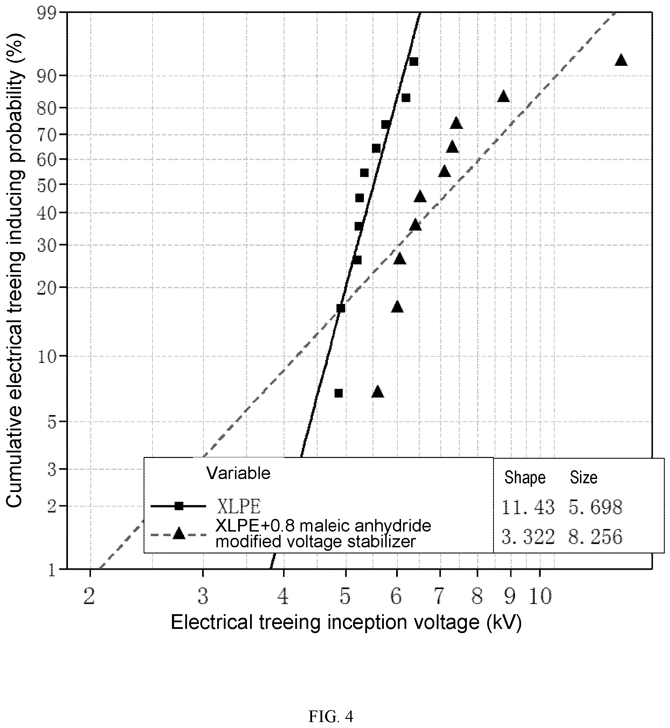

The inception voltages of electrical treeing of the cross-linked polyethylene AC cable insulating materials of Example 7 and Comparative Example 2 were tested, and the test results are shown in FIG. 4.

Test method: The cross-linked polyethylene AC cable insulating materials of Example 7 and Comparative Example 2 were cut into 3mm*10mm*10mm samples. Each sample was placed in a vacuum oven at 80°C for degassing, accelerating precipitation and volatilization of the small molecular components from the sample. After degassing for 135h, the same tungsten needle electrode was inserted into the sample to form a need-plate electrode structure, by using which the inception voltage of electrical treeing of each sample was measured at a 500V/s boosted alternating voltage with a mains frequency. 10 samples were tested for each material, and the Weibull distribution statistical analysis was performed on the test results.

FIG. 4 shows the Weibull distribution plots of the inception voltages of electrical treeing of the cross-linked polyethylene AC cable insulating materials of Example 7 and Comparative Example 2. As can be seen from FIG. 4, the cross-linked polyethylene AC cable insulating material of Example 7 has a significantly higher electrical treeing inception voltage at the same cumulative failure probability, and has a significantly lower electrical treeing inducing probability under the same voltage. The characteristic electrical treeing inception voltage (scale: 8.256kV) is about 44.9% higher than that of the unmodified comparative material (5.698kV). It is thus proved that the maleic anhydride modified voltage stabilizer of the present invention can significantly improve the resistance to electrical treeing. Even through a long-term degassing at a high temperature, the excellent performance of the maleic anhydride modified voltage stabilizer would not be affected. The increase in the electrical treeing inception voltage guarantees the long-term working stability and longer working life of the cross-linked polyethylene AC cable insulating material.

### Effect Example 2

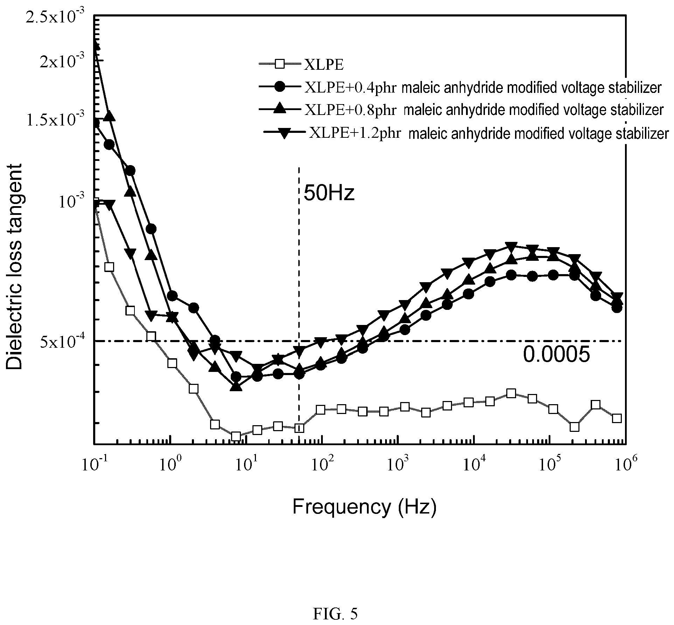

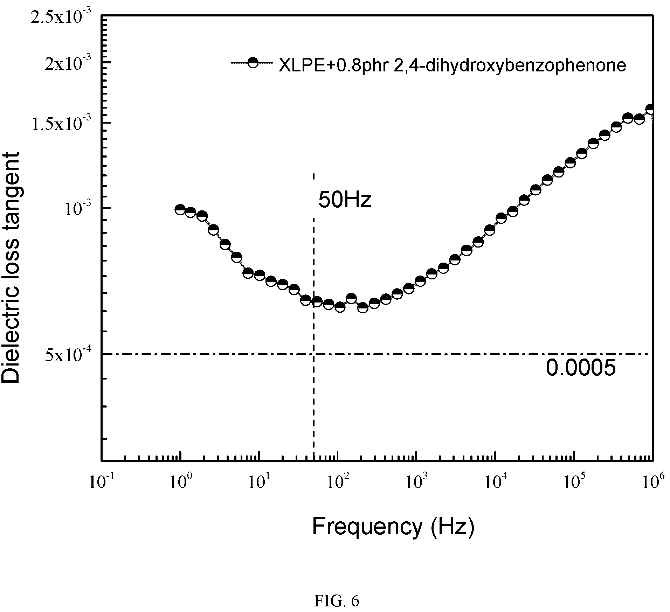

The values of dielectric loss tangent of the cross-linked polyethylene AC cable insulating materials of Examples 7-9 and Comparative Examples 2-3 were tested, and the test results are shown in FIG. 5 and FIG. 6.

Test method: The cross-linked polyethylene AC cable insulating materials of Example 7-9 and Comparative Examples 2-3 were cut into 3mm*10mm*10mm samples, and the values of dielectric loss tangent of the samples at different frequencies were measured.

FIG. 5 shows the values of dielectric loss tangent of the cross-linked polyethylene AC cable insulating materials of Examples 7 to 9 and Comparative Example 2. FIG. 6 shows the values of dielectric loss tangent of the cross-linked polyethylene AC cable insulating material of Comparative Example 3. It can be seen from FIG. 5 and FIG. 6 that the value of dielectric loss tangent of the cross-linked polyethylene AC cable insulating material of Comparative Example 2 at 50 Hz is 0.0002; the value of dielectric loss tangent of the cross-linked polyethylene AC cable insulating material of Comparative Example 3 at 50 Hz is 0.0006; the values of dielectric loss tangent of the cross-linked polyethylene AC cable insulating materials of Example 7, Example 8, and Example 9 at 50 Hz are respectively 0.00042, 0.00043, and 0.00047. The above test results show that the addition of 0.8phr of the traditional voltage stabilizer 2,4-dihydroxybenzophenone significantly increases the value of dielectric loss tangent of the cross-linked polyethylene AC cable insulating material from 0.0002 to 0.0006, exceeding a basic requirement of less than 0.0005 stipulated by a national standard. By using the maleic anhydride modified voltage stabilizer synthesized by the present invention as the voltage stabilizer in the effective addition range (0.4-1.2phr) to increase the inception voltage of the electrical treeing in the cross-linked polyethylene AC cable insulating materials, the values of dielectric loss tangent of the prepared cross-linked polyethylene AC cable insulating materials are all less than the requirement of ≤0.0005 stipulated by the national standard with sufficient margin. Thus, the new voltage stabilizer synthesized by the present invention and the graft-modified cross-linked polyethylene insulating material prepared therefrom have relatively low dielectric loss.

The maleic anhydride modified voltage stabilizer of the present invention has a high polarity carbonyl group in its structure. After the cross-linking reaction between the maleic anhydride modified voltage stabilizer and the polyethylene material, a deep, evenly distributed charge trap is formed. Under the action of the charge trap, the resistivity of the cross-linked polyethylene AC cable insulating material increases, and the leakage current decreases. The dielectric loss of the cross-linked polyethylene AC cable insulating material under an alternating voltage is caused by current leakage on the one hand and by relaxation polarization on the other hand. Although the structure of the maleic anhydride modified voltage stabilizer has the high polarity carbonyl group, which may increase relaxation polarization, the molecular structure of the maleic anhydride modified voltage stabilizer of the present invention has a relatively small contribution to the relaxation polarization behavior at 50Hz but a more obvious inhibitory effect on current leakage, thus decreasing the dielectric loss tangent value of the material at 50Hz, which still meets the requirement of less than 0.0005 at 50Hz stipulated by the standard.

### Effect Example 3

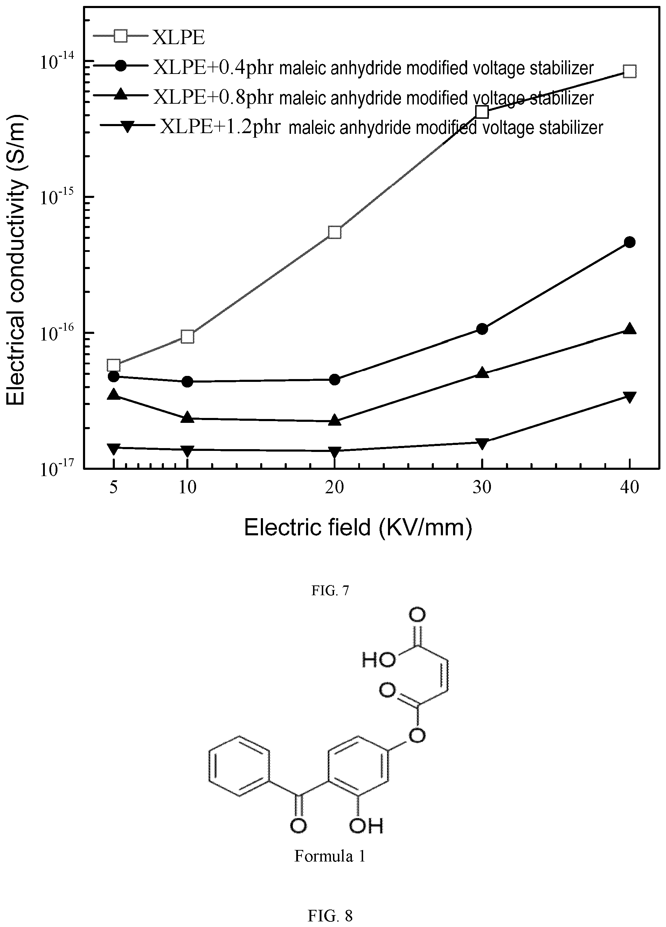

The electrical conductivities of the cross-linked polyethylene AC cable insulating materials of Examples 7-9 and Comparative Example 2 were tested, and the test results are shown in FIG. 7.

Test method: The cross-linked polyethylene AC cable insulating materials of Example 7-9 and Comparative Example 2 were cut into 3mm*10mm*10mm samples, and the electrical conductivities of the samples at different strengths of electric field were measured.

FIG. 7 shows the electrical conductivities of the cross-linked polyethylene AC cable insulating materials of Examples 7-9 and Comparative Example 2 at different strengths of electric field. It can be seen from FIG. 7 that the electrical conductivities of the cross-linked polyethylene AC cable insulating material of Examples 7-9 are significantly lower than that of the cross-linked polyethylene AC cable insulating material of Comparative Example 2; that is, the cross-linked polyethylene AC cable insulating material of the present invention has a higher electrical resistivity, which also reveals that the dielectric loss of the cross-linked polyethylene AC cable insulating material is decreased by the high polarity carbonyl group in the structure of the maleic anhydride modified voltage stabilizer via the mechanism of reducing the leakage current.

Finally, it should be noted that the above embodiments are used to illustrate the technical solutions of the present invention rather than to limit the protection scope of the present invention, which is defined by the appended claims.

## Claims

1. A cross-linked polyethylene AC cable insulating material, comprising following components by mass:
100phr of low-density polyethylene,
0.2-1.2phr of a maleic anhydride modified voltage stabilizer,
0.2-0.5phr of an antioxidant, and
1.5-2.2phr of an initiator;
wherein the low-density polyethylene has a melt index of 1.9-2.1g/10min at 190°C and
2.16kg load and a density of 0.902-0.942g/cm³;
the maleic anhydride modified voltage stabilizer has a structural formula represented by Formula 1:

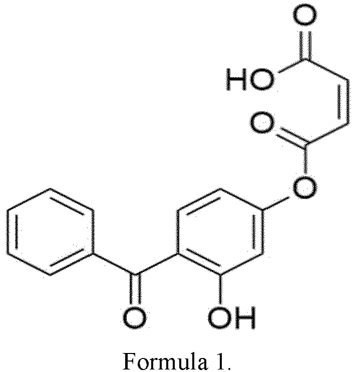

2. The cross-linked polyethylene AC cable insulating material of claim 1, wherein a method for preparing the maleic anhydride modified voltage stabilizer, comprises following steps:
(1) dissolving maleic anhydride and 2,4-dihydroxybenzophenone in tetrahydrofuran to obtain a mixture;
(2) in a protective gas atmosphere, adding a catalyst to the mixture obtained in step (1), stirring, adding water and centrifuging, and then drying an obtained precipitate, thereby achieving the maleic anhydride modified voltage stabilizer;
wherein a molar ratio of 2,4-dihydroxybenzophenone, maleic anhydride, tetrahydrofuran, and catalyst is 2:(1.4-2):(24-125):(0.1-1.2).

3. The cross-linked polyethylene AC cable insulating material of claim 2, wherein the catalyst is concentrated sulfuric acid or *p*-toluenesulfonic acid.

4. The cross-linked polyethylene AC cable insulating material of claim 2, wherein in step (2), stirring time is 6-12h, and stirring temperature is 65-70°C.

5. The cross-linked polyethylene AC cable insulating material of claim 1, wherein the antioxidant is antioxidant 300.

6. The cross-linked polyethylene AC cable insulating material of claim 1, wherein the initiator is dicumyl peroxide.

7. A method for preparing the cross-linked polyethylene AC cable insulating material of any one of claims 1 to 6, comprising following steps:
(1) melting and blending low-density polyethylene, the maleic anhydride modified voltage stabilizer, and an antioxidant together in an extruder, and then extruding and granulating resulting blend, thereby obtaining granules;
(2) adding an initiator melted at 70-80°C to the granules obtained in step (1) to infiltrate the granules, thereby obtaining a polyethylene material;
(3) cross-linking the polyethylene material obtained in step (2) at 150-280°C, 10-20MPa, thereby obtaining the cross-linked polyethylene AC cable insulating material.

## Patentansprüche

1. Vernetztes Polyethylen-AC-Kabelisoliermaterial, umfassend folgende Komponenten in Massenanteilen:
100 phr Polyethylen niedriger Dichte,
0,2-1,2 phr eines mit Maleinsäureanhydrid modifizierten Spannungsstabilisators,
0,2-0,5 phr eines Antioxidanten und
1,5-2,2 phr eines Initiators;
wobei das Polyethylen niedriger Dichte einen Schmelzindex von 1,9-2,1 g/10 min bei 190 °C und einer Belastung von 2,16 kg und eine Dichte von 0,902-0,942 g/cm³ hat;
der mit Maleinsäureanhydrid modifizierte Spannungsstabilisator eine Strukturformel die, die durch Formel 1 repräsentiert wird:

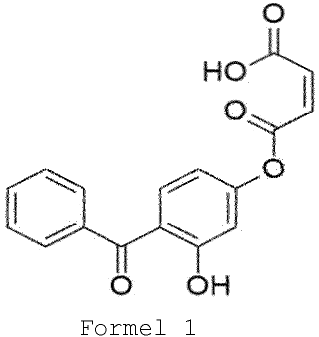

2. Vernetztes Polyethylen-AC-Kabelisoliermaterial gemäß Anspruch 1, wobei ein Verfahren zur Herstellung des mit Maleinsäureanhydrid modifizierten Spannungsstabilisators die folgenden Schritte umfasst:
(1) Auflösen von Maleinsäureanhydrid und 2,4-Dihydroxybenzophenon in Tetrahydrofuran, um eine Mischung zu erhalten;
(2) in einer Schutzgasatmosphäre, Zugabe eines Katalysators zu der in Schritt (1) erhaltenen Mischung, Rühren, Zugabe von Wasser und Zentrifugieren und anschließendes Trocknen eines erhaltenen Niederschlags, wodurch der mit Maleinsäureanhydrid modifizierte Spannungsstabilisator erhalten wird;
wobei ein Molverhältnis von 2,4-Dihydroxybenzophenon, Maleinsäureanhydrid, Tetrahydrofuran und Katalysator 2: (1,4-2): (24-125): (0,1-1,2) ist.

3. Vernetztes Polyethylen-AC-Kabelisoliermaterial gemäß Anspruch 2, wobei der Katalysator konzentrierte Schwefelsäure oder p-Toluolsulfonsäure ist.

4. Vernetztes Polyethylen-AC-Kabelisoliermaterial gemäß Anspruch 2, wobei in Schritt (2) die Rührzeit 6-12 Stunden ist und die Rührtemperatur 65-70 °C ist.

5. Vernetztes Polyethylen-AC-Kabelisoliermaterial gemäß Anspruch 1, wobei der Antioxidant Antioxidant 300 ist.

6. Vernetztes Polyethylen-AC-Kabelisoliermaterial gemäß Anspruch 1, wobei der Initiator Dicumylperoxid ist.

7. Verfahren zur Herstellung des vernetzten Polyethylen-AC-Kabelisoliermaterial gemäß irgendeinem der Ansprüche 1 bis 6, umfassend die folgenden Schritte:
(1) Schmelzen und Mischen von Polyethylen niedriger Dichte, dem mit Maleinsäureanhydrid modifizierten Spannungsstabilisator und einem Antioxidanten in einem Extruder und anschließendes Extrudieren und Granulieren der resultierenden Mischung, wodurch Granulate erhalten werden;
(2) Hinzufügen eines bei 70-80 °C geschmolzenen Initiators zu den in Schritt (1) erhaltenen Granulaten, um die Granulate zu infiltrieren, wodurch ein Polyethylenmaterial erhalten wird;
(3) Vernetzen des in Schritt (2) erhaltenen Polyethylenmaterials bei 150-280 °C, 10-20 MPa, wodurch das vernetzte Polyethylen-AC-Kabelisoliermaterial erhalten wird.

## Revendications

1. Matériau isolant de câble d'alimentation en courant alternatif (CA) en polyéthylène réticulé, comprenant les composants suivants en masse :
100 phr de polyéthylène basse densité,
0,2 à 1,2 phr d'un stabilisateur de tension modifié par anhydride maléique,
0,2 à 0,5 phr d'un antioxydant, et
1,5 à 2,2 phr d'un initiateur ;
dans lequel le polyéthylène basse densité présente un indice de fusion de 1,9 à 2,1 g/10 min à 190°C et une charge de 2,16 kg et une densité de 0,902 à 0,942 g/cm³ ;
le stabilisateur de tension modifié par anhydride maléique présente une formule structurale représentée par la formule 1 :

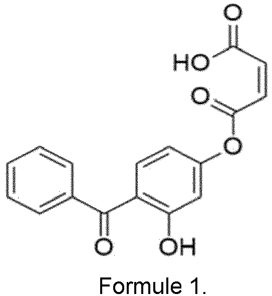

2. Matériau isolant de câble d'alimentation en courant alternatif en polyéthylène réticulé selon la revendication 1, dans lequel un procédé destiné à préparer le stabilisateur de tension modifié par anhydride maléique comprend les étapes suivantes :
(1) dissoudre l'anhydride maléique et la 2,4-dihydroxybenzophénone dans le tétrahydrofurane pour obtenir un mélange ;
(2) dans une atmosphère de gaz protecteur, ajouter un catalyseur au mélange obtenu à l'étape (1), agiter, ajouter de l'eau et centrifuger, puis sécher un précipité obtenu, obtenant ainsi le stabilisateur de tension modifié par anhydride maléique ;
dans lequel un rapport molaire de 2,4-dihydroxybenzophénone, d'anhydride maléique, de tétrahydrofurane, et de catalyseur est 2:(1,4-2):(24-125):(0,1-1,2).

3. Matériau isolant de câble d'alimentation en courant alternatif en polyéthylène réticulé selon la revendication 2, dans lequel le catalyseur est de l'acide sulfurique concentré ou de l'acide p-toluènesulfonique.

4. Matériau isolant de câble d'alimentation en courant alternatif en polyéthylène réticulé selon la revendication 2, dans lequel, à l'étape (2), le temps d'agitation est compris de 6 à 12 h, et la température d'agitation est comprise de 65 à 70°C.

5. Matériau isolant de câble d'alimentation en courant alternatif en polyéthylène réticulé selon la revendication 1, dans lequel l'antioxydant est l'antioxydant 300.

6. Matériau isolant de câble d'alimentation en courant alternatif en polyéthylène réticulé selon la revendication 1, dans lequel l'initiateur est le peroxyde de dicumyle.

7. Procédé destiné à préparer le matériau isolant de câble d'alimentation en courant alternatif (CA) en polyéthylène réticulé selon l'une quelconque des revendications 1 à 6, comprenant les étapes suivantes :
(1) fusionner et mélanger ensemble le polyéthylène basse densité, le stabilisateur de tension modifié par anhydride maléique, et un antioxydant dans une extrudeuse, puis extruder et granuler le mélange résultant, obtenant ainsi des granules ;
(2) ajouter un initiateur fusionné à 70 à 80°C aux granules obtenus à l'étape (1) pour infiltrer les granules, obtenant ainsi un matériau en polyéthylène ;
(3) réticuler le matériau en polyéthylène obtenu à l'étape (2) à 150 à 280°C, 10 à 20 MPa, obtenant ainsi le matériau isolant de câble d'alimentation en courant alternatif en polyéthylène réticulé.
